# EUROPÄISCHE PATENTSCHRIFT

(11) **EP 2 595 752 B1**
(45) Veröffentlichungstag und Bekanntmachung des Hinweises auf die Patenterteilung: **22.08.2018**
(21) Anmeldenummer: 11734112.3
(22) Anmeldetag: 21.07.2011
(51) Int. Cl.: B01L 3/00, G01N 33/543

(54) **VERFAHREN ZUR HYDROPHILISIERUNG VON OBERFLÄCHEN FLUIDISCHER KOMPONENTEN UND ANALYSATOR MIT FLUIDPACKS FÜR DAS VERFAHREN**
METHOD FOR HYDROPHILISING SURFACES OF FLUID COMPONENTS AND ANALYSATOR WITH FLUID PACKS USING THE METHOD
PROCÉDÉ D'HYDROPHILISATION DE SURFACES DE COMPOSANTS FLUIDIQUES ET APPAREIL D'ANALYSE AVEC DES RESERVOIRS POUR LE PROCÉDÉ

(30) Priorität: 23.07.2010 EP 10170613
(43) Veröffentlichungstag der Anmeldung: 29.05.2013
(73) Patentinhaber: F. Hoffmann-La Roche AG, 4070 Basel (CH); Roche Diagnostics GmbH, 68305 Mannheim (DE)
(72) Erfinder: HOFMANN, Wolfgang, A-8055 Graz (AT); NOORMOFIDI, Taghi, A-8054 Graz (AT); ZAHRL, Doris, A-8020 Graz (AT)
(74) Vertreter: Babeluk, Michael
(86) Internationale Anmeldenummer: PCT/EP2011/062506
(87) Internationale Veröffentlichungsnummer: WO 2012/010653

(56) Entgegenhaltungen:
- EP-A1- 2 067 848
- WO-A1-01/47637
- WO-A1-2009/062940
- WO-A2-2006/127451
- US-A1- 2009 130 746

## Beschreibung

Die Erfindung betrifft ein Verfahren zur zumindest teilweisen Belegung eines zumindest ein Sensorelement aufweisenden Messkanals einer in einen Analysator einsetzbaren Sensorkassette mit einem hydrophilen Polymer. Weiters betrifft die Erfindung einen Analysator mit einem in den Analysator austauschbar einsetzbaren Fluidpack, das zumindest ein Behältnis mit Wasch-, Kalibrier- und/oder Qualitätskontrollflüssigkeiten für den Analysator aufweist, sowie mit einer in den Analysator austauschbar einsetzbaren Sensorkassette mit einem zumindest ein Sensorelement aufweisenden Messkanal.

Insbesondere bei der Bestimmung von gasförmigen Analyten (O₂, CO₂) in wässrigen Flüssigkeiten können Probleme bei der Probenmessung bzw. bei der Kalibrierung oder Qualitätskontrolle auftreten, wenn die Probe bzw. das Qualitätskontroll- oder Kalibriermittel den flüssigkeitsführenden Bereich des sensorischen Elements nur unvollständig füllt oder wenn sich dort Gasblasen, beispielsweise Luftblasen, befinden. Gasblasen bilden sich insbesondere bei Vorliegen von uneinheitlichen inneren Oberflächen innerhalb des Messkanals der Sensorkassette, welche unterschiedliche Benetzungseigenschaften durch Flüssigkeiten aufweisen. Besonders häufig kommt es an den Stellen des Messkanals zur Bildung oder Festsetzung von Gasblasen, an welchen ein sprunghafter Übergang in den Benetzungseigenschaften der inneren Oberflächen der verschiedenen fluidischen Komponenten bzw. Bauteile vorhanden ist. Dies ist beispielsweise dann der Fall, wenn Oberflächen aus unterschiedlichen Materialien aufeinander stoßen. Der Messkanal besteht im Allgemeinen aus vielen einzelnen fluidischen Bauteilen aus unterschiedlichen Materialien, deren aneinander grenzende Oberflächen sich durch eine unterschiedliche Hydrophilie oder Hydrophobie auszeichnen und infolge dessen unterschiedliche Benetzungseigenschaften besitzen.

In der US 4,358,423 A (Nedetzky) wird bereits auf das Problem von eingeschlossenen Luftblasen hingewiesen, welche das Messresultat verfälschen, da die Luftblasen eine ausreichende Benetzung der Oberfläche der jeweils eingesetzten Sensorelemente verhindern. Maßnahmen, die einen derartigen Fehler erkennen, sind insbesondere bei automatisch arbeitenden Analysatoren notwendig, bei welchen der Füllvorgang der Messkapillare bzw. die Blasenfreiheit der Probe in der Messkammer automatisch kontrolliert werden muss. Zur Lösung des Problems wird hier ein Verfahren vorgeschlagen, bei welchem der Wert des elektrischen Widerstandes zwischen wenigstens zwei Stellen in der Messkammer gemessen wird, wobei der Füllvorgang der Messkammer in Abhängigkeit der festgestellten Größe des gemessenen Widerstandes gesteuert wird.

Die EP 0 379 156 beschreibt Beschichtungsverfahren, bei welchen zunächst auf die Oberfläche einer medizinischen Vorrichtung, insbesondere eines Katheders, eine Polyisocyanatlösung aufgebracht wird, diese (optional) eingetrocknet wird und anschließend eine weitere Lösung eines carboxylsäurehaltigen Polymers aufgebracht wird. Bei solchen zwei- oder mehrstufigen Verfahren mit mehreren beteiligten Reagenzienlösungen und chemischen Reaktionsschritten sind meist viele Prozessschritte notwendig, die anwenderseitig nicht oder nur mit großem Aufwand durchführbar sind.

Eine Beschichtung von Oberflächen medizinischer Implantate, Katheter und Herzschrittmacher mit chitosanhaltigen Schichten ist beispielsweise aus der US 5,578,073 A bekannt, erfolgt hier jedoch zur Verringerung des Thromboserisikos beim Einbringen dieser medizintechnischen Vorrichtungen in den menschlichen Körper. Die Schicht besteht aus Chitosan und einer zusätzlichen, biologisch aktiven Komponente, beispielsweise PVA oder aus Serumalbumin, welches in eine Chitosanmembran eingebettet ist. Derartige Schichten sind allerdings für Messkanäle mit Sensorelementen ungeeignet.

Die US 4,752,426 beschreibt Verfahren zur Hydrophilisierung von Oberflächen, bei welchen zunächst mittels einer Niedertemperatur-Plasmabehandlung chemisch reaktive Gruppen bzw. Radikale auf der Oberfläche erzeugt werden. Anschließend wird eine Monomer-Lösung auf diese Oberfläche aufgebracht. Die Monomere reagieren dort chemisch mit den chemisch reaktiven Gruppen bzw. Radikalen auf der Oberfläche, sodass letztlich auf der Oberfläche mittels Pfropfpolymerisation eine Beschichtung gebildet wird. Nachteilig bei diesen Verfahren ist, dass diese Verfahrensschritte und -bedingungen werkseitig durchgeführt und möglichst exakt aufeinander abgestimmt werden müssen. Beispielsweise müssen die Plasmabehandlungsparameter derartig gewählt werden, dass möglichst nur solche chemisch reaktive Gruppen bzw. Radikale auf der Oberfläche gebildet werden, welche auch als Polymerisationskeime für die nachfolgende Pfropfpolymerisation dienen können.

In der EP 1 595 605 B1 erfolgt die Lösung des Benetzungsproblems durch Bereitstellung eines fluidischen Systems (z.B. eine Sensorkassette) für einen Analysator, welches ein oder mehrere fluidische Bauteile (z.B. Messkanal) und zumindest ein Sensorelement enthält, auf deren inneren Oberflächen direkt ohne weitere Zwischenschichten ein Film eines hydrophilen Polymers aufgebracht wird. Dabei wird zunächst die innere Oberfläche des fluidischen Systems einer physikalischchemischen Vorbehandlung unterzogen. Anschließend werden die inneren Oberflächen der Bauteile mit einer Lösung desselben hydrophilen Polymers in Kontakt gebracht und danach die Lösung durch ein gasförmiges Medium ersetzt, wobei die Oberflächen mit einem Teil der Lösung benetzt bleiben. Schließlich entsteht auf den inneren Oberflächen ein Film des hydrophilen Polymers durch Entzug des Lösungsmittels. Das relativ aufwändige Beschichtungsverfahren kann nur werkseitig durchgeführt werden.

Aus der US 2009/130746 A1 ist ein werkseitiges Verfahren zur Beschichtung der inneren Oberfläche eines Mikrokanalsystems bekannt, um nicht-spezifische Absorptionen von Reagenzien bei PCR-Analyseverfahren zu vermeiden. Dabei werden Lösungen eingesetzt, die Chitosan oder Chitosanderivate enthalten. Das Mikrokanalsystem der Chips wird mit einer 5%igen Chitosanlösung gefüllt und mit Mineralöl abgedeckt. Danach folgt eine zwölfstündige Hitzebehandlung bei 75°C. Nach der Entfernung der Chitosanlösung werden die Mikrokanäle mit einem Lösungsmittel, Wasser und einer Pufferlösung gespült. Ein derartiges Verfahren kann bei Messkanälen, die Sensorelemente enthalten, nicht angewandt werden.

In vielen Anwendungsfällen wäre es allerdings von Vorteil, keine ausschließlich werkseitige Beschichtung bzw. Hydrophilisierung der inneren Oberflächen des Messkanals durchzuführen, da zur Vermeidung von Alterungseinflüssen die Hydrophilisierung erst kurz vor (oder während) der tatsächlichen Verwendung der Kassette in einem Analysator erfolgen soll. Bei einer werkseitigen Verwendung von wässrigen Lösungen zur Oberflächenbehandlung kann es zudem zu unerwünscht frühzeitigen Reaktionen einzelner Sensorelemente mit der wässrigen Lösung kommen, z.B. einer Aktivierung des Sensorelementes durch Aufnahme von Wasser ("wet up" des Sensors).

Für eine ausreichende Hydrophilisierung wäre auch der Einsatz eines hochwirksamen Detergens in einer der Betriebsflüssigkeiten denkbar. Obwohl die prinzipielle Wirksamkeit dieser Maßnahme bewiesen ist, ist deren Einsatz im Rahmen der vorliegenden Erfindung aber aufgrund von Nebeneffekten nicht angebracht.

Die Aufgabe der Erfindung besteht in der Bereitstellung einer Sensorkassette mit hydrophilisierten Oberflächen des Messkanals zwecks Vermeidung von Gasblasenbildung bzw. Anhaftung von Gasblasen bei Befüllung mit wässrigen Betriebsflüssigkeiten oder Probenflüssigkeiten, wobei die Hydrophilisierung anwenderseitig kurz vor oder während des Gebrauchs der im Analysator vorliegenden Sensorkassette erfolgen soll.

Diese Aufgabe wird erfindungsgemäß dadurch gelöst,
a. dass eine Sensorkassette in den Analysator eingesetzt wird,
b. dass eine wässrige Lösung in den Messkanal der Sensorkassette eingeleitet wird, die einen pH-Wert <7 aufweist und Chitosan oder ein Chitosanderivat enthält, wobei das Chitosan oder das Chitosanderivat einen Deacetylierungsgrad von 10% bis 100%, bevorzugt von 50% bis 100%, besonders bevorzugt von 70% bis 95% aufweist, sowie, sowie
c. dass die Chitosan oder ein Chitosanderivat enthaltende wässrige Lösung nach einer kurzen Verweilzeit von 5 s bis 30 s durch ein flüssiges Medium ersetzt wird, das im Vergleich zur wässrigen Lösung, die Chitosan oder ein Chitosanderivat enthält, einen höheren pH-Wert aufweist, wobei Reste des Chitosans oder Chitosanderivats auf der inneren Oberfläche des Messkanals (5) und auf den den Messkanal (5) begrenzenden Teilen des zumindest einen Sensorelementes verbleiben und die innere Oberfläche und die den Messkanal (5) begrenzenden Teile hydrophilisieren.

Das Verfahren kann auf einfache Weise anwenderseitig - ohne jede Vorbehandlung - bevorzugt als automatische Routine des Analysators durchgeführt werden.

Zur Erhöhung der Hydrophilisierung können die Schritte b. und c. bevorzugt auch mehrfach hintereinander durchgeführt werden.

Weiters ist es erfindungsgemäß möglich, bereits in Verwendung stehende Sensorkassetten in vorgegebenen Zeitabständen oder nach einer vorgegebenen Anzahl von Analytbestimmungen und/oder Kalibrations- oder Qualitätskontrollvorgängen automatisch oder manuell ausgelöst den Schritten b. und c. zu unterwerfen, um die Hydrophilisierung der Kassette wieder aufzufrischen.

Gegenstand der Erfindung ist auch die Verwendung von Chitosan oder einem Chitosanderivat zur Herstellung eines inselartigen oder vollflächigen Belags auf inneren Oberflächen der fluidischen Komponenten von Sensorkassetten, insbesondere eines Messkanals, der zumindest ein Sensorelement aufweist. Überraschenderweise hat sich gezeigt, dass ein Chitosanbelag auf den Sensorelementen die Sensorfunktion nicht beeinträchtigt, insbesondere dann, wenn ein inselartiger Belag oder ein Monolayer vorliegen. Die mit dem Lumen des Messkanals in Verbindung stehenden Teile der Sensorelemente bilden einen integralen Bestandteil der Wandung dieses Messkanals und werden mit dem erfindungsgemäßen Verfahren zusammen mit den anderen Teilen des Messkanals ebenfalls mit einem Chitosanbelag beschichtet. Wenn im Rahmen der vorliegenden Patentanmeldung der einfacheren Lesbarkeit halber von Messkanal gesprochen wird, sind hiermit erfindungsgemäß auch die oben beschrieben mit dem Lumen des Messkanals in Verbindung stehenden Teile der Sensorelemente mit umfasst, welche einen integralen Bestandteil der Wandung dieses Messkanals bilden. Es hat sich weiterhin gezeigt, dass auch bereits ein nicht flächendeckender, inselförmiger Belag eine erfindungsgemäße Verbesserung der hydrophilen Oberflächeneigenschaften bewirkt.

Als Sensorelemente werden im Sinne der vorliegenden Anmeldung alle Vorrichtungen angesehen, mit welchen physikalische oder chemische Parameter einer Flüssigkeit bestimmt werden können und welche direkt mit der zu untersuchenden Probe in Kontakt treten. Solche Sensoren können beispielsweise elektrochemische oder optische Sensoren zur Bestimmung der Gaswerte, des pH-Wertes, der Ionenwerte und der Metabolitwerte von Blutproben sein. Sensorelemente bestehen üblicherweise aus einer oder mehreren Schichten organischer und/oder anorganischer, vorzugsweise polymerer, Substanzen, welche auf einem hiervon unterschiedlichen Trägersubstrat auf der der Probenflüssigkeit zugewandten Seite eines Messkanals angebracht sind. Die Erfindung wird im Folgenden anhand von Ausführungsbeispielen näher erläutert. Die nachfolgenden Angaben sind großteils auf die Verwendung einer Chitosanlösung zur Hydrophilisierung abgestellt.

Chitosan ist ein Polyaminosaccharid, welches sich vom Chitin ableitet. Liegen im Gesamtmolekül im erhöhten Maße deacetylierte 2-Amino-2-desoxy-β-D-glukopy-ranose-Einheiten vor, spricht man von Chitosan. Es ergibt sich ein lineares Polymer, das üblicherweise aus etwa 2000 Monomeren besteht. Es sind jedoch auch Chitosane mit weniger oder mehr Monomereinheiten bekannt, welche ebenfalls im Rahmen der vorliegenden Erfindung mitumfasst sind.

Der pKs-Wert (auch pKa-Wert genannt) eines exemplarischen Chitosans liegt bei ca. 6,5, bedingt durch die pH-abhängige Protonierung bzw. Deprotonierung der Aminogruppen des Chitosanmoleküls. Das bedeutet, dass bei niedrigeren pH-Werten (unterhalb des pKs-Wertes) die überwiegende Anzahl der Aminogruppen positiv geladen und daher das Chitosanmolekül gut wasserlöslich ist (Polykation). Daraus erklärt sich, dass Chitosan in Säuren bevorzugt löslich ist.

Bei höheren pH-Werten nimmt der Anteil der protonierten Aminogruppen ab. Das bedeutet, dass bei pH-Werten oberhalb des pKs-Wertes des Chitosans die überwiegende Anzahl der Aminogruppen ungeladen vorliegt und daher das Chitosanmolekül weniger gut wasserlöslich ist.

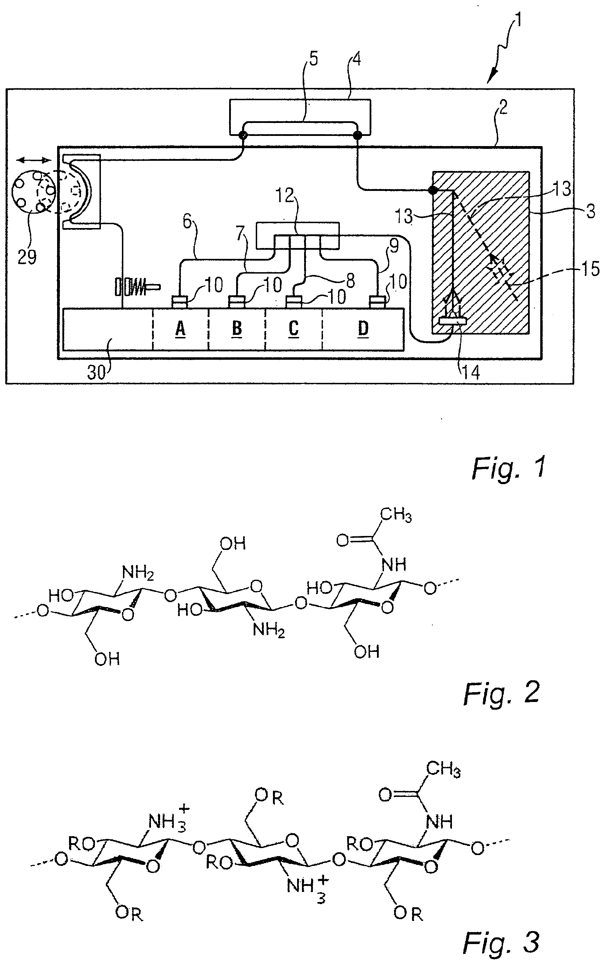

Figur 2 zeigt einen typischen Ausschnitt aus der Strukturformel eines Chitosanmoleküls. Das hier dargestellte Chitosan liegt im deprotonierten Zustand vor, d.h. die Aminogruppen liegen im ungeladenen Zustand vor. Dieser Zustand tritt vor allem bei erhöhten pH-Werten auf.

Die pH-Abhängigkeit der Chitosan-Löslichkeit wirkt sich positiv auf die Abscheidung des Chitosans aus, da die Substitution der schwach sauren Chitosanlösung im Messkanal durch eine Betriebslösung mit schwach alkalischen pH-Wert eine leichte Alkalisierung bewirkt, die sich positiv auf die Abscheidung des Polysaccharids auswirkt.

Neben den reinen Chitosanen können auch Chitosanderivate in Rahmen der vorliegenden Erfindung eingesetzt werden.

Solche erfindungsgemäßen Chitosanderivate sind Derivate des Chitosans, bei welchen die Wasserstoffatome der OH-Gruppen zumindest teilweise durch niedermolekulare Substituenten ersetzt sind. Mögliche Substituenten sind im Rahmen der vorliegenden Erfindung bevorzugt -CH₃ (Methyl), -CH₂-CH₃ (Ethyl),-CH₂-CH₂-CH₃ (n-Propyl), -CH(CH₃)₂ (iso-Propyl) -CH₂-O-CH₃ (Methoxymethyl) und -CH₂-O-CH₂-O-CH₃, -CH₂-CH₂-OH (Hydroxyethyl), -CO-CH₃ (Acetyl). Hierbei können sowohl alle Wasserstoffatome der OH-Gruppen durch einen oder mehrere dieser Substituenten ersetzt sein oder auch nur ein Teil der Wasserstoffatome der OH-Gruppen des Chitosans.

Chitosanderivate im Sinne der vorliegenden Anmeldung sind in Fig. 3 dargestellt.

Fig. 3 zeigt einen typischen Ausschnitt aus der Strukturformel eines Chitosans (für den Fall, dass alle Reste R Wasserstoffatome sind) oder Chitosanderivate, bei welchem jedes R für zum Teil unterschiedliche oder gleichartige, niedermolekulare Substituenten aus einer Gruppe umfassend -H, -CH₃, -CH₂-CH₃, -CH₂-CH₂-CH₃, -CH(CH₃)₂, -CH₂-O-CH₃, und -CH₂-O-CH₂-O-CH₃, -CH₂-CH₂-OH, -CO-CH₃ steht. Das hier dargestellte Chitosanderivat liegt hier im protonierten Zustand vor, d.h. die Aminogruppen liegen als positiv geladene -NH₃⁺-Gruppen vor. Dieser Zustand tritt vor allem bei erniedrigten pH-Werten auf.

Wenn im Rahmen der vorliegenden Patentanmeldung der einfacheren Lesbarkeit halber von Chitosanen oder Chitosanlösungen gesprochen wird, sind hiermit erfindungsgemäß auch die oben beschrieben Chitosanderivate und Lösungen dieser Chitosanderivate mit umfasst.

Es zeigen:
- Fig. 1: einen Analysator mit einem eingesetzten Fluidpack (Reagenzienkassette) mit mehreren Behältnissen zur Aufnahme der Betriebsflüssigkeiten und einer Chitosanlösung in einer schematischen Darstellung;
- Fig. 2: die Strukturformel von Chitosan;
- Fig. 3: die Strukturformel von Chitosanderivaten im Sinne der vorliegenden Anmeldung;
- Fig. 4: ein Zustandsdiagramm einer Chitosanlösung, wobei auf der Abszisse der pH-Wert und auf der Ordinate der Chitosangehalt der Lösung aufgetragen ist; sowie
- Fig. 5: eine Messanordnung zur Bestimmung des Benetzungsverhaltens einer Substratoberfläche in schematischer Darstellung.

Der in Fig. 1 schematisch dargestellte Analysator 1 zur Analyse von medizinischen Probenflüssigkeiten, beispielsweise von Blutproben, weist eine Reagenzienkassette bzw. ein Fluidpack 2 auf, das austauschbar in den Analysator 1 einsetzbar ist. Im Fluidpack 2 sind mehrere als Reagenzienbeutel ausgeführte Behältnisse A bis D angeordnet, welche Betriebsflüssigkeiten, wie beispielsweise Kalibrier-, Qualitätskontroll-, und Waschflüssigkeiten beinhalten, die wahlweise einer Eingabeeinrichtung 3 und in weiterer Folge einem in einer Sensorkassette 4 angeordneten Messkanal 5 mit zumindest einem Sensorelement zugeführt werden können. Die Eingabeeinrichtung 3 des Analysators 1 weist ein verschwenkbares Eingabeelement 13 (beispielsweise eine Hohlnadel) auf, das in einer Grundposition mit einem Andockelement 14 zur Zufuhr von Kalibier- und Waschmittel in Verbindung steht, wobei in einer aus der Grundposition ausgeschwenkten Stellung 15 Probenflüssigkeiten zugeführt werden können. Die Probeneingabe kann aus unterschiedlichen Gefäßen (z.B. Spritze, Kapillare, Glasgefäß, etc.) erfolgen.

Im vorliegenden Beispiel enthält eine der Wasch-, Kalibrier- und/oder Qualitätskontrollflüssigkeiten in den Behältnissen A bis C zusätzlich Chitosan, oder das Fluidpack 2 weist ein weiteres Behältnis D zur Aufnahme einer vorzugsweise wässrigen Chitosanlösung auf. Diese Ausführungsform ist besonders bevorzugt, da hierdurch das erfindungsgemäße Verfahren automatisiert durchgeführt werden kann. Alternativ ist es auch möglich, die Chitosanlösung auch anderweitig in den Messkanal der Sensorkassette einzuleiten, beispielsweise durch Ansaugen einer chitosanhaltigen Lösung durch das Eingabeelement 13 des Analysators.

Jeder Reagenzienbeutel A bis D weist direkt bei der Einmündung der jeweiligen Anschlussleitung 6, 7, 8, 9 ein vom Analysator ansteuerbares Mehrwegeventil 10 (Beutelventil) mit zumindest zwei Ventilstellungen auf, wobei die erste Ventilstellung eine Fluidverbindung zwischen der jeweiligen Anschlussleitung 6, 7, 8, 9 und dem zugehörigen Reagenzienbeutel A bis D herstellt. In der zweiten Ventilstellung wird der jeweilige Reagenzienbeutel A bis D verschlossen und ein Zugang zur Umgebungsluft hergestellt. Alle von den Mehrwegeventilen 10 wegführenden Anschlussleitungen 6, 7, 8, 9 der Reagenzienbeutel A bis D münden in eine gemeinsame Sammelleitung 12, welche eine Verbindung zum Andockelement 14 der Probeneingabeeinrichtung 3 herstellt.

Die Fluidleitung führt nach der Sensorkassette 4 über den feststehenden Teil einer im Analysator 1 integrierten Schlauchpumpe 29 und mündet in einen in der Reagenzienkassette 2 angeordneten Abfallbeutel 30.

Zusammenfassend stehen somit folgende Lösungen zur Verfügung:
- die Bereitstellung einer chitosanhaltigen Lösung, bevorzugt mit physiologischem pH-Wert, vorzugsweise im (ohnehin vorhandenen) Fluidpack des Analysators
- die Bereitstellung eines Verfahrens zur zumindest teilweisen Belegung der inneren Oberflächen des Messkanals mit Chitosan kurz vor oder während des Gebrauchs der Sensorkassette.
- die Bereitstellung einer Sensorkassette wobei der Messkanal oder andere fluidische Komponenten der Sensorkassette zwecks Hydrophilisierung an deren inneren Oberflächen einen inselartigen oder vollflächigen Belag aufweisen, der Chitosan enthält oder aus Chitosan besteht. Gleiches gilt für die entsprechenden Chitosanderivate.

Der Messkanal wird zunächst mit einer wässrigen Lösung von Chitosan befüllt und anschließend durch eine Lösung ersetzt, die einen im Vergleich zur Chitosanlösung höheren pH-Wert aufweist, z.B. eine schwach alkalische Betriebsflüssigkeit (Kalibrier-, Qualitätskontroll- oder Waschflüssigkeit) des Analysators. Durch die pH-Erhöhung werden die protonierten Aminogruppen von Chitosan deprotoniert, wodurch sich die Zahl der positiven Ladungen verringert. Die dadurch bewirkte verringerte Löslichkeit von Chitosan begünstigt die Ablagerung an den inneren Oberflächen des Messkanals.

Alternativ kann die chitosanhaltige Lösung auch als Betriebsflüssigkeit (Kalibrierflüssigkeit, QC-Flüssigkeit, Waschflüssigkeit etc.) ausgeführt sein, indem den bestehenden Inhaltsstoffen der Betriebsflüssigkeit noch Chitosan (und evtl. optional noch weitere Substanzen, wie Puffersubstanzen) zugesetzt wird.

Die wässrige Chitosanlösung weist vorzugsweise einen pH-Wert zwischen 6,4 und 6,8 auf.

Die derart behandelten Oberflächen des Messkanals weisen für wässrige Lösungen eine Oberflächenbenetzbarkeit auf, die für alle den Messkanal bildenden Oberflächen verbessert wird, und höher ist als die Oberflächenbenetzbarkeit der inneren Oberflächen des Messkanals ohne das Vorhandensein des Chitosanbelags. Durch die verbesserte und vereinheitlichte Benetzbarkeit für wässrige Flüssigkeiten ist das Risiko von Gasblasenbildung oder -festsetzung während des Befüllvorgangs mit Betriebs- und Messflüssigkeiten erheblich vermindert.

### Experimentelle Ergebnisse:

### Herstellung der Chitosanlösung:

Die Herstellung der Chitosanlösung erfolgt werkseitig nach folgendem Schema:
- Auflösen von Chitosan in Säure
- Einstellen auf einen bestimmten pH-Wert mit Hilfe von Puffersubstanzen

Als saure und basische Komponenten kommen Mineralsäuren und alle Basen in Frage, wobei zur Etablierung eines Puffersystems organische HEPES-Base (als Hepes-Na Salz) vorteilhaft ist.

Als Chitosan-Rohstoff wird das Produkt Sigma #448877 (Sigma-Aldrich, eingesetzt, spezifiziert mit einem Deacetylierungsgrad zwischen 75% und 100%. Das Produkt wird weiters durch rheologische Parameter spezifiziert.

Im nachfolgenden Beispiel wurde ein Chitosan verwendet, welches gemäß Hersteller mit einem Deacetylierungsgrad von 82% und einem mittleres Molekulargewicht von 300 - 400 kDa spezifiziert war.

Als wirksam hat sich beispielsweise folgende in Tabelle 1 dargestellte Rezeptur erwiesen (die angegebenen Konzentrationen sind die Konzentrationen der finalen Lösung im Endvolumen):

**Tabelle 1:**

| | |
|---|---|
| Chitosan (Sigma #448877, 82 % Deacetylierungsgrad, 300-400 kDa) | ca.0,3 g/l |
| NaCl | 49 mmol/l |
| Hepes, freie Säure | 15 mmol/l |
| Hepes, Natriumsalz | 3 mmol/l |

Weiters kann die Lösung ein Konservierungsmittel enthalten.

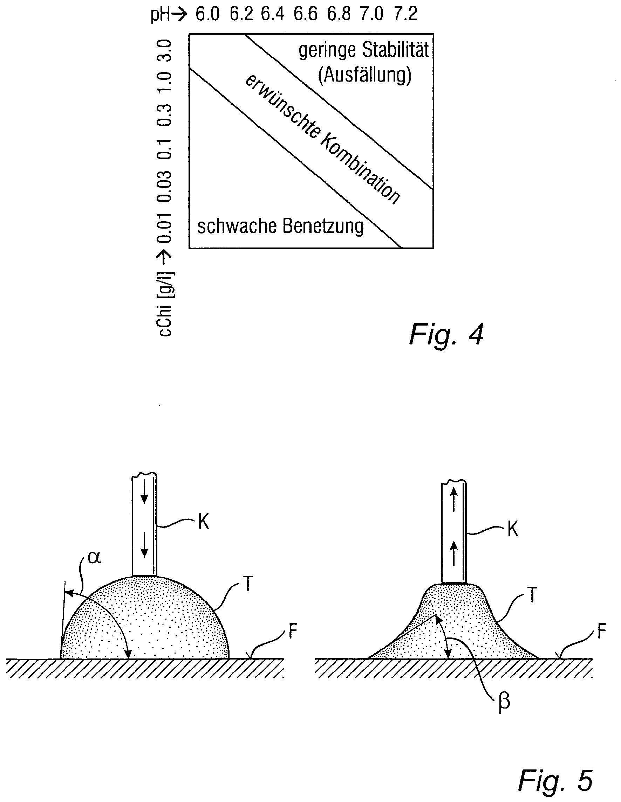

Der pH-Wert dieser Lösung liegt z.B. bei 6,7. Bei der Rezepturerstellung hat sich herausgestellt, dass die Eignung der Lösung nicht nur von der Chitosankonzentration, sondern auch stark vom eingestellten pH-Wert abhängt. Bei hohem pH-Wert wird die Lösung instabil aufgrund von Ausfällung des Polymers. Bei zu niedrigem pH-Wert der Lösung kommt es nur zu einer unzureichenden Belegung der inneren Oberflächen. Die Abhängigkeiten entsprechen sinngemäß dem in Fig. 4 dargestellten Schema, wobei auf der Abszisse der pH-Wert und auf der Ordinate der Chitosangehalt aufgetragen ist.

Die Chitosan Lösung befindet sich bevorzugt im Reagenzienpack 2 des Analysators, und zwar in einem beutelartigen Behältnis D gemäß Fig. 1 (siehe auch EP 2 077 452 A1)

Die im Behältnis D des Fluidpacks 2 vorliegende Chitosanlösung wird durch einen automatisierten Ablauf in den Messkanal 5 der Sensorkassette 4 gesaugt und verbleibt dort für eine kurze Verweilzeit von beispielsweise 5 bis 30 s, wobei die inneren Oberflächen des Messkanals 5 mit Chitosan zumindest teilweise belegt werden.

Entweder wird nun der Messkanal 5 leer gesaugt und die Sensorkassette ist dann (nach einer gewissen Trocknungszeit) bereit für die bestimmungsgemäße Verwendung, oder die Chitosanlösung wird direkt durch eine der Betriebsflüssigkeiten mit einem etwas höheren pH-Wert ersetzt, wobei es aufgrund der laminaren Strömung zu einer schrittweisen Vermischung der beiden Lösungen kommt. Im Rahmen der vorliegenden Erfindung wurde überraschend erkannt, dass sich diese Vermischung aufgrund des steigenden pH-Wertes positiv auf die Abscheidung des Chitosan an den inneren Oberflächen des Messkanals auswirkt.

Das Verfahren zur zumindest teilweisen Belegung der inneren Oberflächen des Messkanals der Sensorkassette mit Chitosan wird vor dem erstmaligen Gebrauch bzw. Aktivierung einer Sensorkassette durchgeführt.

Weiters ist auch vorgesehen, das Verfahren während der 'in-use' Lebensdauer der Sensorkassette wiederholt durchzuführen, um die zumindest teilweise Belegung der inneren Oberflächen des Messkanals der Sensorkassette mit Chitosan aufrecht zu erhalten bzw. zu erneuern.

Es hat sich gezeigt, dass die Benetzungswirkung von Chitosan zumindest für einen gewissen Zeitraum persistent ist. Die Deposition des Polysaccharides bewirkt eine zumindest zeitlich begrenzte Hydrophilisierung der Oberflächen des Sensorkanals.

Die in der folgenden Tabelle 2 zusammengefassten Daten zeigen die Wirkung von Benetzungslösungen in einem beispielhaften Messsystem. Dabei ist ein Probenkanal mit einem Sensorarray (siehe beispielsweise WO 2009/062940) eingesetzt. Zwischen den Sensoren befinden sich Metallkontakte, die im Normalfall dazu dienen, aufgrund elektrischer Kontaktierung Proben zu erkennen. Das System kann auch dazu herangezogen werden, um Luftblasen innerhalb des Messkanals zu erkennen (siehe US 4,358,423).

Im Experiment wurden neue, nicht verwendete Sensorkassetten erstmalig eingesetzt. Bei diesen Kassetten ist der Sensorkanal trocken und wurde noch nicht mit einer Flüssigkeit in Kontakt gebracht. Es folgt eine Erstbefüllung des Sensorkanals mit einer Testflüssigkeit. Als Testflüssigkeit wurden verwendet: eine Betriebsflüssigkeit (A/B), Blut (C) oder eine Chitosanlösung (D).

Die eingesetzte Betriebsflüssigkeit dient als Kalibrierflüssigkeit und ist eine wässrige Lösung von Salzen und Puffersubstanzen mit eingestellten Konzentrationen an Gasen (O₂/CO₂) und einem pH-Wert von 7,35. Das Monitoring der Gasblasen erfolgt bei der nachfolgenden abwechselnden Befüllung und Entleerung des Sensorkanals mit Betriebsflüssigkeit.

**Tabelle 2:**

| **Experiment** | **A** | **B** | **C** | **D** |
|---|---|---|---|---|
| **Erstbenetzung** | Erstbenetzung durch Kalibrationslösung | Erstbenetzung durch Kalibrationslösung mit Tensid Triton X 100 | Erstbenetzung durch Blut | Erstbenetzung durch Chitosanlösung pH 6.7 (Sigma #448877, 82 % Deacetylierungsgrad, 300-400 kDa) |
| **Betriebsflüssigkeit** | Kalibrationslösung | Kalibrationslösung mit Tensid Triton X 100 | Kalibrationslösung | Kalibrationslösung |
| **Anzahl der beobachteten Zyklen** | 2071 | 1055 | 829 | 832 |
| **Erkannte Luftblasen** | 43% | 6% | 20% | 3% |

Wie aus den Prozentzahlen hervorgeht, zeigt der Tensidzusatz zur Kalibrationslösung eine drastische Reduktion der Luftblasen (Experiment B) (der Tensidzusatz ist aber in der eingesetzten Konzentration nicht realisierbar). Auch die Erstbenetzung mit Blut hat einen positiven Effekt (Experiment C) auf die folgenden Befüllvorgange mit Betriebsflüssigkeit. Dieser Effekt ist bekannt und wahrscheinlich auf die Deposition bestimmter Proteine zurückzuführen. Die Erstbenetzung mit der Chitosanlösung (Experiment D) hat aufgrund der Polymerdeposition eine deutliche Reduktion der Luftblasen zufolge, Die Frequenz der Luftblasenbildung ist ähnlich niedrig, wie bei Experiment B, obwohl die Betriebsflüssigkeit in Experiment D kein Tensid enthält.

### Effekte unterschiedlicher Chitosane auf Benetzungsverhalten von Oberflächen mit Wasser:

Die Wirkung der Behandlung einer Substratoberfläche mit einer Chitosanlösung kann beispielsweise durch deren Benetzungsverhalten mit Wasser messtechnisch erfasst werden. Dazu wird der Grenzwinkel eines Wassertropfens auf der Oberfläche vermessen, und zwar im Vorrück-Modus und im Rückzugs-Modus. Fig. 5 verdeutlicht die beiden Verfahren. Während im Vorrück-Modus (siehe linke Seite) der "advancing angle" a, also der augenblicklich bei Flüssigkeitszugabe durch eine Kapillare K sich einstellende Randwinkel des Tropfens T zur Oberfläche F als Maßzahl ermittelt wird, wird im Rückzugs-Modus der Tropfen T teilweise aufgesaugt und das Beharrungsvermögen der Flüssigkeit auf der Oberfläche F durch den "receding angle" β ermittelt. Bei den mit Chitosanlösung behandelten Oberflächen zeigen sich große Differenzen zwischen dem Vorrück- und Rückzugswinkel, ein Verhalten das als Kontaktwinkelhysterese bezeichnet wird und als Abweichung vom idealen thermodynamischen Verhalten mit Inhomogenitäten der Oberflächenenergien erklärt wird.

Es ist daher anzunehmen, dass bei der beschriebenen Behandlung eines Messkanals mit einer Chitosanlösung die Belegung der zunächst hydrophoben Kanaloberflächen nur teilweise und domänen- bzw. inselartig erfolgt. Indes ist der Messkanal mit den Sensorelementen während des Betriebs des Analysators fast immer mit Flüssigkeit gefüllt und damit ist das Rückzugsverhalten wichtiger für die Benetzung der Sensoren als das Vorrückverhalten. Bei der folgenden Charakterisierung unterschiedlicher Chitosane wurde ausschließlich der Rückzugswinkel β betrachtet.

Versuchsbeschreibung: Die Substrate (Oberfläche Polyethylen) werden in die Chitosan enthaltende, wässrige Lösung 30 Sekunden eingelegt. Danach wird die Lösung mit einer wässrigen Pufferlösung (pH 7,3) abgewaschen. Nach dem Abtrocknen der Flüssigkeit werden die Oberflächen einem Benetzungswinkeltest unterzogen. Eingesetztes Messgerät: Dataphysics® Contact Angle Systems OCA. Der Benetzungswinkel wird im Rückzugsverfahren ermittelt. Es werden 7 µl destilliertes Wasser aufdispensiert und davon 5 µl abgesaugt. Der Rückzugswinkel β des verbleibenden Tropfens T wird durch Bildverarbeitung ermittelt.

Die einzelnen Chitosantypen sind charakterisiert durch den Deacetylierungsgrad und durch die Molekulargewichtsverteilung. Da die Molekulargewichtsverteilung nur sehr aufwendig ermittelbar ist, wird bei den technischen Chitosanprodukten auch auf ein standardisiertes rheologisches Verfahren (Viskosität einer einprozentigen Lösung in Essigsäure) zurückgegriffen. Der Viskositätswert in mPas wird als Kennzahl herangezogen und ist ein indirektes Maß für das Molekulargewichtsmittel.

Die folgende Tabelle 4 zeigt für verschiedene Chitosane die Reduktion des Rückzugswinkels β nach dem Benetzungsverfahren. Als Referenzwerte wurden die Werte nach Benetzung mit chitosanfreiem Puffer herangezogen. Als Spezifikationen sind die Deacetylierungsgrade und die Molekulargewichtsbereiche angegeben. Die angegebenen Molekulargewichtsbereiche sind laut Hersteller typisch für das Molmassenmittel des jeweilige Produkt.

**Tabelle 4:**

| Deacetylierungsgrad | Molekulargewichtsbereich | Hersteller/Produktnummer | Rückzugswinkel β auf Polyethylen |
|---|---|---|---|
| [%] | [kDa] | | [°] |
| 95 | 50 - 100 | Heppe*/24701 | 38,9 |
| 95 | 300 - 400 | Heppe*/24706 | 40,5 |
| 95 | 600 - 800 | Heppe*/24711 | 48,1 |
| 82 | 300 - 400 | Sigma**/448877 | 48,7 |
| 70 | 300 - 400 | Heppe*/24206 | 50,7 |
| Substrat (Polyethylen) nach Benetzung mit Chitosan-freier Lösung | | | 95,4 |

| | | | |
|---|---|---|---|
| *: Hersteller Heppe Medical Chitosan GmbH, Halle a. d. Saale, Deutschland **: Dieses Chitosan ist das in den vorherigen Beispielen verwendete Chitosan Sigma #448877 mit einem herstellerseitig spezifizierten Deacetylierungsgrad von 82% und einem mittleren Molekulargewicht von 300 - 400 kDa | | | |

Folgende Tabelle 5 zeigt die Wirkung der Benetzungsprozedur auf verschiedenen Materialien, die im Sensorkanal typischerweise eingesetzt werden. Für die Experimente wurde eine der obigen Benetzungslösungen eingesetzt, und zwar die Lösung mit Chitosan Sigma #448877 mit einem herstellerseitig spezifizierten Deacetylierungsgrad von 82% und einem mittleren Molekulargewicht von 300 - 400 kDa.

**Tabelle 5:**

| Materialoberfläche | Rückzugswinkel β nach Behandlung mit Chitosan-freier Lösung | Rückzugswinkel β nach Behandlung mit Chitosanlösung |
|---|---|---|
| Polyethylene | 94,5° | 48,1° |
| Barex® | 68,0° | 12,2° |
| Borosilikatglas | 52,5° | < 10° |
| Polycarbonat | 65,5° | 25,9° |
| Polyurethan | 83,1° | 27,5° |

Bei Barex® handelt es sich um ein Acrylonitrile-Methyl Acrylate Copolymer (ANMA) der INEOS USA LLC in Delaware City US (www.ineosbarex.com).

Es ist damit gezeigt, dass bevorzugt Chitosane im Molekulargewichtsbereich von 50 - 800 kDa und Deacetylierungsgraden zwischen 70% und 95% wirksam sind, und dass sich die Benetzung auf unterschiedlichen Materialien durch die Benetzungsprozedur verbessert. Aber auch Chitosane bzw. Chitosanderivate mit niedrigeren bzw. höheren mittleren Molekulargewichten oder niedrigeren bzw. höheren Deactylierungsgraden können jedoch im Rahmen der vorliegenden Erfindung eingesetzt werden. So kann der Molekulargewichtsbereich zwischen 1 kDa und 5.000 kDa liegen, der Deactylierungsgrad zwischen 10% und 100%.

## Patentansprüche

1. Verfahren zur zumindest teilweisen Belegung der inneren Oberflächen eines zumindest ein Sensorelement aufweisenden Messkanals (5) einer in einen Analysator (1) austauschbar einsetzbaren Sensorkassette (4) mit einem hydrophilen Polymer, **dadurch gekennzeichnet,**
a. **dass** eine Sensorkassette (4) in den Analysator (1) eingesetzt wird,
b. **dass** eine wässrige Lösung in den Messkanal (5) der Sensorkassette (4) eingeleitet wird, die einen pH-Wert <7 aufweist und Chitosan oder ein Chitosanderivat enthält, wobei das Chitosan oder das Chitosanderivat einen Deacetylierungsgrad von 10% bis 100%, bevorzugt von 50% bis 100%, besonders bevorzugt von 70% bis 95% aufweist, sowie
c. **dass** die Chitosan oder ein Chitosanderivat enthaltende wässrige Lösung nach einer kurzen Verweilzeit von 5 s bis 30 s durch ein flüssiges Medium ersetzt wird, das im Vergleich zur wässrigen Lösung, die Chitosan oder ein Chitosanderivat enthält, einen höheren pH-Wert aufweist, wobei Reste des Chitosans oder Chitosanderivats auf der inneren Oberfläche des Messkanals (5) und auf den den Messkanal (5) begrenzenden Teilen des zumindest einen Sensorelementes verbleiben und die innere Oberfläche und die den Messkanal (5) begrenzenden Teile hydrophilisieren.

2. Verfahren nach Anspruch 1, **dadurch gekennzeichnet, dass** die Schritte b. und c. zur Erhöhung der Hydrophilisierung mehrfach hintereinander durchgeführt werden.

3. Verfahren nach Anspruch 1 oder 2, **dadurch gekennzeichnet, dass** bereits in Verwendung stehende Sensorkassetten (4) in vorgegebenen Zeitabständen den Schritten b. und c. unterworfen werden.

4. Verfahren nach einem der Ansprüche 1 bis 3, **dadurch gekennzeichnet, dass** die wässrige Lösung, die Chitosan oder ein Chitosanderivat enthält, einen pH-Wert zwischen 6,4 und 6,8, aufweist.

5. Verfahren nach Anspruch 1, **dadurch gekennzeichnet, dass** als flüssiges Medium zum Verdrängen der wässrigen Lösung, die Chitosan oder ein Chitosanderivat enthält, eine bevorzugt schwach alkalische Betriebsflüssigkeit des Analysators, beispielsweise eine Wasch-, Kalibrier- oder Qualitätskontrollflüssigkeit, verwendet wird.

6. Verfahren nach einem der Ansprüche 1 bis 5, **dadurch gekennzeichnet, dass** die wässrige Lösung, die Chitosan oder ein Chitosanderivat enthält, aus einem in den Analysator (1) austauschbar einsetzbaren Fluidpack (2) entnommen wird, welches die Betriebsflüssigkeiten, beispielsweise eine Wasch-, Kalibrier- und/oder Qualitätskontrollflüssigkeit, für den Analysator (1) enthält.

7. Analysator (1) mit einem in den Analysator austauschbar einsetzbaren Fluidpack (2) mit mehreren Behältnissen (A, B, C, D) zur Aufnahme der Betriebsflüssigkeiten, das zumindest ein Behältnis (A, B, C) mit Wasch-, Kalibrier- und/oder Qualitätskontrollflüssigkeiten aufweist, sowie mit einer in den Analysator austauschbar einsetzbaren Sensorkassette (4) mit einem zumindest ein Sensorelement aufweisenden Messkanal (5), **dadurch gekennzeichnet, dass** eine der Wasch-, Kalibrier- und/oder Qualitätskontrollflüssigkeiten des Fluidpacks (2) oder eine wässrige Lösung in einem separaten Behältnis (D) des Fluidpacks (2) zusätzlich Chitosan oder ein Chitosanderivat enthält, wobei das Chitosan oder das Chitosanderivat einen Deacetylierungsgrad von 10% bis 100%, bevorzugt von 50% bis 100%, besonders bevorzugt von 70% bis 95% aufweist, und das die im Fluidpack (2) vorliegende Chitosanlösung durch einen automatisierten Ablauf in den Messkanal (5) der Sensorkassette (4) absaugbar ist und dort für eine kurze Verweilzeit von 5 bis 30 s bringbar ist.

8. Analysator (1) nach Anspruch 7, **dadurch gekennzeichnet, dass** die Konzentration des Chitosans oder des Chitosanderivats in der Wasch-, Kalibrier- und/oder Qualitätskontrollflüssigkeit oder der wässrigen Lösung in dem separaten Behältnis zwischen 0,03 g/l und 3 g/l, vorzugsweise bei ca. 0,3 g/l, liegt.

## Claims

1. Method for at least partially applying a hydrophilic polymer to the inner surfaces of a measurement channel (5) of a sensor cartridge (4), which is to be replaceably inserted into an analyzer (1), said measurement channel (5) comprising at least one sensor element, **characterised in that**
(a) a sensor cartridge (4) is inserted into the analyzer (1),
(b) an aqueous solution is entered into the measurement channel (5) of the sensor cartridge (4), said solution having a pH-value <7 and containing chitosan or a chitosan derivative, wherein the chitosan or chitosan derivative has a degree of deacetylation from 10% to 100%, and preferably from 50% to 100%, and especially preferably from 70% to 95%, and that
(c) the aqueous solution containing said chitosan or chitosan derivative is replaced after a short residence time of 5 sec to 30 sec by a liquid medium, which in comparison with the aqueous solution containing chitosan or chitosan derivative has a higher pH-value, wherein residues of the chitosan or chitosan derivative remain on the inner surface of the measurement channel (5) and on parts of the at least one sensor element bounding the measurement channel (5), and hydrophilize the inner surface and the parts bounding the measurement channel (5).

2. Method according to claim 1, **characterised in that** steps (b) and (c) are repeatedly executed one after the other to improve hydrophilization.

3. Method according to claim 1 or 2, **characterised in that** sensor cartridges (4) which are already in use are treated at predetermined intervals of time according to steps (b) and (c).

4. Method according to any of claims 1 to 3, **characterised in that** the aqueous solution containing chitosan or a chitosan derivative has a pH-value between 6.4 and 6.8.

5. Method according to claim 1, **characterised in that** the liquid medium used in replacing the aqueous solution containing chitosan or a chitosan derivative is a preferably weak alkaline operational fluid of the analyzer, for instance a rinsing, calibrating or quality control fluid.

6. Method according to any of claims 1 to 5, **characterised in that** the aqueous solution containing chitosan or a chitosan derivative is taken from a fluidpack (2) that can be replaceably inserted into the analyzer (1), which contains operational fluids for the analyzer (1), for instance a rinsing, calibrating and/or quality control fluid.

7. Analyzer (1), comprising a fluidpack (2), which can be replaceably inserted into an analyzer (1) and has several containers (A, B, C, D) for receiving operational fluids, which comprises at least one container (A, B, C) rinsing, calibrating and/or quality control fluids, and comprising a sensor cartridge (4) which can be replaceably inserted into an analyzer (1) and comprises at least one measurement channel (5) having at least one sensor element, **characterised in that** one of the rinsing, calibrating and/or quality control fluids of the fluidpack (2) or an aqueous solution additionally contains chitosan or a chitosan derivative in a separate container (D) of the fluidpack (2), said chitosan or chitosan derivative having a degree of deacetylation of 10% to 100%, preferably 50% to 100%, especially preferably 70% to 95%, and that the chitosan solution present in the fluidpack (2) can be extracted by suction by automatic sequence into the measurement channel (5) of the sensor cartridge (4) and can be brought there for a short residence time of 5 to 30 s.

8. Analyzer (1) according to claim 7, **characterised in that** the concentration of the chitosan or chitosan derivative in the rinsing, calibrating and/or quality control fluid or the aqueous solution in the separate container is between 0.03g/l and 3g/l, preferably about 0.3g/l.

## Revendications

1. Procédé pour le revêtement au moins partiel des surfaces internes d'un canal de mesure (5), présentant au moins un élément de détection, d'une cassette de détection (4) pouvant être placée de manière échangeable dans un analyseur (1), présentant un polymère hydrophile, **caractérisé**
a. **en ce que** la cassette de détection (4) est placée dans l'analyseur (1),
b. **en ce qu'**une solution aqueuse est introduite dans le canal de mesure (5) de la cassette de détection (4), qui présente un pH < 7 et qui contient du chitosane ou un dérivé de chitosane, le chitosane ou le dérivé de chitosane présentant un degré de désacétylation de 10% à 100%, de préférence de 50% à 100%, de manière particulièrement préférée de 70% à 95%, et
c. **en ce que** la solution aqueuse contenant du chitosane ou un dérivé de chitosane est remplacée après un temps de séjour court de 5 s à 30 s par un milieu liquide qui, par rapport à la solution aqueuse qui contient du chitosane ou un dérivé de chitosane, présente un pH plus élevé, les restes du chitosane ou du dérivé de chitosane restant sur la surface interne du canal de mesure (5) et sur les parties délimitant le canal de mesure (5) dudit au moins un élément de détection et hydrophilisant la surface interne et les parties délimitant le canal de mesure (5).

2. Procédé selon la revendication 1, **caractérisé en ce que** les étapes b. et c. sont réalisées plusieurs fois successivement pour augmenter l'hydrophilisation.

3. Procédé selon la revendication 1 ou 2, **caractérisé en ce que** des cassettes de détection (4) déjà en cours d'utilisation sont soumises aux étapes b. et c à des intervalles définis dans le temps.

4. Procédé selon l'une quelconque des revendications 1 à 3, **caractérisé en ce que** la solution aqueuse, qui contient du chitosane ou un dérivé de chitosane, présente un pH entre 6,4 et 6,8.

5. Procédé selon la revendication 1, **caractérisé en ce qu'**on utilise un liquide de fonctionnement de préférence faiblement alcalin, par exemple un liquide de lavage, d'étalonnage ou de contrôle de la qualité, de l'analyseur comme milieu liquide pour chasser la solution aqueuse qui contient du chitosane ou un dérivé de chitosane.

6. Procédé selon l'une quelconque des revendications 1 à 5, **caractérisé en ce que** la solution aqueuse qui contient du chitosane ou un dérivé de chitosane est prélevée d'un Fluidpack (2), utilisé de manière interchangeable dans l'analyseur (1), qui contient les liquides de fonctionnement, par exemple un liquide de lavage, d'étalonnage et/ou de contrôle de la qualité, pour l'analyseur (1).

7. Analyseur (1) présentant un Fluidpack (2) pouvant être utilisé de manière interchangeable dans l'analyseur, présentant plusieurs récipients (A, B, C, D) pour recevoir les liquides de fonctionnement, qui présente au moins un récipient (A, B, C) présentant des liquides de lavage, d'étalonnage et/ou de contrôle de la qualité, ainsi qu'une cassette de détection (4) pouvant être utilisée de manière interchangeable dans l'analyseur, présentant un canal de mesure (5) présentant au moins un élément de détection, **caractérisé en ce qu'**un des liquides de lavage, d'étalonnage et/ou de contrôle de la qualité du Fluidpack (2) ou une solution aqueuse dans un récipient séparé (D) du Fluidpack (2) contient en outre du chitosane ou un dérivé de chitosane, le chitosane ou le dérivé de chitosane présentant un degré de désacétylation de 10% à 100%, de préférence de 50% à 100%, de manière particulièrement préférée de 70% à 95% et la solution de chitosane se trouvant dans le Fluidpack (2) pouvant être aspirée par un fonctionnement automatisé dans le canal de mesure (5) de la cassette de détection (4) et pouvant y être amenée pour un court temps de séjour de 5 à 30 secondes.

8. Analyseur (1) selon la revendication 7, **caractérisé en ce que** la concentration en chitosane ou en dérivé de chitosane dans le liquide de lavage, d'étalonnage et/ou de contrôle de la qualité ou dans la solution aqueuse dans le récipient séparé se situe entre 0,03 g/l et 3 g/l, de préférence à environ 0,3 g/l.
